# EUROPÄISCHE PATENTSCHRIFT

(11) **EP 0 957 119 B1**
(45) Veröffentlichungstag und Bekanntmachung des Hinweises auf die Patenterteilung: **20.07.2005**
(21) Anmeldenummer: 99108455.9
(22) Anmeldetag: 12.05.1999
(51) Int. Cl.: C08G 18/08, C08G 18/12, A61K 7/06

(54) **Vernetzte, wasserlösliche oder wasserdispergierbare Polyurethane**
Cross-linked, water-soluble or water-dispersible polyurethanes
Polyuréthanes réticulés, solubles ou dispersables dans l'eau

(30) Priorität: 14.05.1998 DE 19821732
(43) Veröffentlichungstag der Anmeldung: 17.11.1999
(73) Patentinhaber: BASF AKTIENGESELLSCHAFT, 67056 Ludwigshafen (DE)
(72) Erfinder: Nguyen Kim, Son, 69502 Hemsbach (DE); Sanner, Axel, 67227 Frankenthal (DE); Hössel, Peter, 67105 Schifferstadt (DE); Schehlmann, Volker, 67354 Römerberg (DE)
(74) Vertreter: Kinzebach, Werner, Dr.

(56) Entgegenhaltungen:
- EP-A- 0 593 959
- EP-A- 0 741 156
- EP-A- 0 773 246
- DATABASE WPI Section Ch, Week 9349 Derwent Publications Ltd., London, GB; Class A14, AN 93-392731 XP002111768 & JP 05 295078 A (DAINIPPON INK & CHEM KK) , 9. November 1993 (1993-11-09)

## Beschreibung

Die vorliegende Erfindung betrifft vernetzte, wasserlösliche oder wasserdispergierbare Polyurethane aus mindestens einem Polyurethanpräpolymer mit endständigen Isocyanatgruppen und mindestens einem Polymerisat mit gegenüber Isocyanatgruppen reaktiven Gruppen, die ausgewählt sind unter Hydroxyl-, primären und sekundären Amino- und/oder Carboxylgruppen.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 15°C) besitzen.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Ein weiterer aktueller Anspruch an Haarbehandlungsmittel ist es, dem Haar ein natürliches Aussehen und Glanz zu verleihen, z. B. auch dann, wenn es sich um von Natur aus besonders kräftiges und/oder dunkles Haar handelt.

Die DE-A-42 25 045 und die WO 94/03515 beschreiben unvernetzte, wasserlösliche oder in Wasser dispergierbare Polyurethane, die mindestens 5 Mol-% eines Polykondensats aus Milchsäure und einem Polyol einpolymerisiert enthalten, sowie die Verwendung von wasserlöslichen oder wasserdispergierbaren Polyurethanen aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat,
als Hilfsmittel in kosmetischen und pharmazeutischen Zubereitungen.

Die DE-A-42 41 118 beschreibt die Verwendung von unvernetzten, kationischen Polyurethanen und Polyharnstoffen als Hilfsmittel in kosmetischen und pharmazeutischen Zubereitungen.

Die EP-A-619 111 beschreibt die Verwendung von Polyurethanen auf Basis von organischen Diisocyanaten, Diolen und 2,2-Hydroxymethyl-substituierten Carboxylaten der Formel 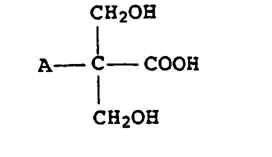 worin A für ein Wasserstoffatom oder eine C₁-C₂₀-Alkylgruppe steht, in Haarfixierungsmitteln. Zumindest ein Teil der Carbonsäuregruppen wird dabei mit einer organischen oder anorganischen Base neutralisiert. Filme auf Basis dieser linearen Polyurethane weisen eine mangelnde Flexibilität auf und sind daher verbesserungswürdig.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethan- und/oder Polyharnstoffeinheit mit anionischen oder kationischen Gruppen umfasst. Vernetzte, wasserlösliche oder wasserdispergierbare Polyurethane sind nicht beschrieben. Die WO 97/25021 hat einen vergleichbaren Offenbarungsgehalt. Diese kosmetischen Mittel eignen sich unter anderem zur Behandlung von keratinischen Materialien. Die Auswaschbarkeit dieser Filmbildner ist jedoch nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser lösliches oder dispergierbares Salz der Formel I:

[A-(X)ₙ]ⁿ⁻ · [HₘB]^{m+} I

worin
- A: für einen kosmetisch akzeptablen aliphatischen, cycloaliphatischen oder aromatischen Rest steht, der siloxan- und/oder fluorhaltige Einheiten aufweisen kann,
- X: für eine Carboxylat-, Sulfonat-, Phosphat- oder Phosphonatgruppe steht;
- B: eine kosmetisch akzeptable Aminbase bedeutet die siloxan- und/oder fluorhaltige Einheiten aufweisen kann;
- n: für 1 bis 30 steht; und
- m: die Wertigkeit des Amins B bedeutet.

Haarspray-Formulierungen auf Basis dieser siloxanhaltiger Salze führen zu Filmen, die leicht, z. B. durch mechanische Belastung, von der Haaroberfläche abgelöst werden. Die Festigungswirkung dieser Formulierungen ist daher verbesserungswürdig.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt. Haarspray auf Basis dieser Polyurethane sind noch verbesserungswürdig. Insbesondere bei der Formulierung von Haarsprays mit einem hohen Treibgasgehalt und/oder einem hohen Gehalt an organischen Lösungsmitteln und gegebenenfalls gleichzeitigem Einsatz von Sprühzerstäubern zur Erzielung sehr kleiner Tröpfchen können Probleme auftreten.

Die EP-A-0 389 386 beschreibt lineare Diorganopolysiloxan-Polyester-Blockcopolymere mit eingebauten Urethaneinheiten, die sich zur kontrollierten Freigabe pharmazeutischer Zubereitungen eignen.

Die EP-A-0 626 432 beschreibt hitzehärtbare Überzugsmittel aus einem Polyesteroligomer-Polyacrylat mit freien OH-Gruppen und mindestens einem Polyisocyanat mit freien NCO-Gruppen und deren Verwendung zur Herstellung von Klarlackschichten. Diese Polymerisate sind nach ihrer Vernetzung weder wasserlöslich noch wasserdispergierbar und eignen sich nicht zur Herstellung von kosmetischen Zubereitungen.

Die WO 97/00664 beschreibt eine wässrige Nagelpolitur, die ein bifunktionelles Urethanacrylat-Oligomer, welches mit einem Acrylharz vernetzt ist, umfasst. Polyurethane aus mindestens einem Polyurethanpräpolymer mit endständigen Isocyanatgruppen werden nicht beschrieben. Die aus diesen Zusammensetzungen resultierenden Filme sind wasserbeständig und nicht redispergierbar. Sie eignen sich nicht in kosmetischen Zusammensetzungen zur Anwendung auf der Haut oder auf den Haaren.

Die EP-A-0 687 459 beschreibt Haarbehandlungsmittel auf Basis einer wässrigen Polymerdispersion, die erhältlich ist durch radikalische Pfropfcopolymerisation eines monoethylenisch ungesättigten Siloxanmakromonomers und wenigstens eines Polyurethan- und/oder Polyharnstoff-Copolymers. Vernetzte Polyurethane aus Polyurethanpräpolymeren mit endständigen Isocyanatgruppen und Polymerisaten mit gegenüber Isocyanatgruppen reaktiven Gruppen sind in diesem Dokument nicht beschrieben.

Die US-A-3,927,199 beschreibt eine Haarfestiger-Zusammensetzung auf Basis eines Copolymers, welches (1) 30 bis 60 Gew.-% eines N-(C₂- bis C₁₂)-Alkyl(meth)acrylamids, (2) 12 bis 18 Gew.-% eines α,β-ethylenisch ungesättigten, säuregruppenhaltigen Comonomers, (3) 20 bis 55 Gew.-% wenigstens eines Comonomers, das ausgewählt ist unter Alkyl(meth)acrylaten, Hydroxyalkyl(meth)acrylaten, Vinylacetat, Vinylpropionat, Diacetonacrylamid, Styrol und α-Methylstyrol, einpolymerisiert enthält. Nach Neutralisation mit einer Base wird ein Copolymer erhalten, welches in Wasser löslich oder dispergierbar ist und sich zur Formulierung von Haarsprays eignet. Vernetzte Polyurethane auf Basis von Polyurethanpräpolymeren mit endständigen Isocyanatgruppen sind in diesem Dokument nicht beschrieben. Diese Haarfestiger zeigen in wässrigen Formulierungen einen starken Anstieg der Viskosität mit zunehmendem Wassergehalt und eignen sich nicht zur Herstellung VOC-armer Produkte.

Die WO 97/23519 beschreibt anionische aminogruppenhaltige Polymerisate, die durch radikalische Polymerisation olefinischer aminogruppenhaltiger Monomere in wässrigem Medium in Gegenwart eines Azoinitiators erhältlich sind. Dabei weist der Initiator neutralisierte Carboxylgruppen auf. Als aminogruppenhaltige Monomere werden z. B. Amino(meth)acrylate, wie tert.-Butylaminoethyl(meth)acrylat eingesetzt. Die Polymerisate können weitere olefinisch ungesättigte Monomere, wie (Meth)acrylsäureester und Hydroxyalkyl(meth)acrylate einpolymerisiert enthalten. Sie eignen sich zur Herstellung von Ein- oder Zweikomponenten-Beschichtungsmitteln, für die Beschichtung von Metall- und Plastikoberflächen, die durch Vernetzung mit Polyisocyanaten oder Polyepoxiden gehärtet werden. Vernetzte, wasserlösliche oder wasserdispergierbare Polymerisate, die sich zum Einsatz als oder in einem kosmetischen Mittel eignen, werden nicht beschrieben. Polymerisate, die ein Aminoalkyl(meth)acrylat in einer Menge von 0,1 bis 10 Gew.-% einpolymerisiert enthalten und deren Verwendung als Zwischenprodukte zur Herstellung von vernetzten, wasserlöslichen oder wasserdispergierbaren Polyurethanen sind ebenfalls nicht beschrieben.

Die WO 97/23527 beschreibt ein wässriges Einkomponenten-Beschichtungsmittel auf Basis eines Polyharnstoffbinders aus einem Polymerisat mit gegenüber Isocyanatgruppen reaktiven primären und/oder sekundären Aminogruppen, welches zusätzlich Hydroxylgruppen aufweisen kann, und einem Polyisocyanat. Die aminogruppenhaltigen Polymerisate enthalten dabei (1) wenigstens ein olefinisch ungesättigtes Monomer mit primären oder sekundären Aminogruppen, z. B. ein Aminoalkyl(meth)acrylat, (2) gegebenenfalls ein hydroxylgruppenhaltiges Monomer, z. B. ein Hydroxyalkyl(meth)acrylat, (3) gegebenenfalls weitere olefinisch ungesättigte Monomere, ausgewählt unter (Meth)acrylsäureestern, Styrol, Alkylstyrolen, (Meth)acrylnitril, Vinylacetat etc. einpolymerisiert. Die durch Vernetzung mit dem Polyurethan resultierenden Bindemittel eignen sich zur Beschichtung von Metall- und Plastikoberflächen. Vernetzte, wasserlösliche oder wasserdispergierbare Polyurethane sind nicht beschrieben. Polymerisate, die 0,1 bis 10 Gew.-% wenigstens eines Monomers mit einer primären und/oder sekundären Aminogruppe einpolymerisiert enthalten und dessen Verwendung als Zwischenprodukte zur Herstellung vernetzter, wasserlöslicher oder wasserdispergierbarer Polyurethane sind ebenfalls nicht beschrieben.

Die US-A-5,612,404 hat einen der WO 97/23519 und WO 97/23527 entsprechenden Offenbarungsgehalt.

Die WO 96/30425 beschreibt ein wässriges Zweikomponenten-Polyisocyanat-Beschichtungsmittel auf Basis eines im Wesentlichen isocyanatgruppenfreien Emulgators, umfassend ein Reaktionsprodukt aus: (1) einem Isocyanat und (2) einer Komponente, die ausgewählt ist unter hydroxylgruppenhaltigen Polyalkylethern mit mindestens 5 Ethylenoxideinheiten, davon verschiedenen Alkoholen, Aminoverbindungen und Mischungen davon. Dabei können als Bindemittel mit gegenüber Isocyanatgruppen reaktiven Gruppen die in der WO 97/23527 beschriebenen Polymerisate auf Basis von Aminoalkyl(meth)acrylaten eingesetzt werden.

Die WO 97/22632 beschreibt ein Verfahren zur Herstellung einer Klebstoffzusammensetzung, umfassend:
(a) Umsetzung eines Diols oder Polyols mit einem Diisocyanat zu einem Präpolymer mit terminalen Isocyanatgruppen;
(b) Umsetzung des ersten Präpolymers zur Überführung wenigstens eines Teils der terminalen Isocyanatgruppen in terminale Methacrylatgruppen, z. B. mit einem Aminoalkyl(meth)acrylat, wie N-tert.-Butylaminoethylmethacrylat;
(c) Umsetzung der restlichen Isocyanatgruppen mit einem Abstopper, vorzugsweise einem Alkanolamin, wie Triethanolamin;
(d) thermische oder photochemische Härtung des Präpolymers aus (c) unter Ausbildung eines Polyurethan(meth)acrylat-basierenden Klebemittels. Die resultierenden Klebemittel sind drucksensitiv und eignen sich für die Anwendung auf der Haut, z. B. in medizinischen Produkten für die Wundversorgung.

Vernetzte, wasserlösliche oder wasserdispergierbare Polyurethane aus einem Polyurethanpräpolymer und einem Polymerisat mit gegenüber Isocyanatgruppen reaktiven Gruppen sind in diesem Dokument nicht beschrieben.

Die EP-A-0 773 246 beschreibt wasserlösliche oder wasserdispergierbare Pfropfpolymere aus
A) einem in Wasser löslichen oder dispergierbaren Polyurethanpräpolymer mit endständigen Isocyanatgruppen und
B) einem freie Aminogruppen enthaltenden Protein.

Diese eignen sich als Hilfsmittel in der Kosmetik und insbesondere als Haarfestiger mit verbesserter Auswaschbarkeit. Nachteilig am Einsatz der Proteine ist, dass diese eine Stabilisierung durch Konservierungsmittel erfordern und als Naturstoffe häufig schwankende Produkteigenschaften aufweisen.

Der vorliegenden Erfindung liegt die Aufgabe zugrunde, neue Polyurethane, insbesondere für Haarbehandlungsmittel, zur Verfügung zu stellen, die einerseits als Haarfestiger brauchbar sind, andererseits aber auch eine gute Auswaschbarkeit (Redispergierbarkeit) besitzen. Vorzugsweise sollen sie glatte und flexible Filme bilden.

Überraschenderweise wurde nun gefunden, dass diese Aufgabe durch vernetzte, wasserlösliche bzw. durch Wasser dispergierbare Polyurethane gelöst wird, welche das Umsetzungsprodukt aus mindestens einem Polyurethanpräpolymer mit endständigen Isocyanatgruppen und aus mindestens einem Polymerisat mit gegenüber Isocyanatgruppen reaktiven Gruppen sind.

Gegenstand der vorliegenden Erfindung sind daher kosmetische oder pharmazeutische Mittel gemäß Anspruch 1, Polyurethane gemäß Anspruch 8 und deren Verwendung.

Bei der Komponente a) handelt es sich bevorzugt um Diole, Diamine, Aminoalkohole, und Mischungen davon. Das Molekulargewicht dieser Verbindungen liegt vorzugsweise in einem Bereich von etwa 56 bis 280. Gewünschtenfalls können bis zu 3 Mol-% der genannten Verbindungen durch Triole oder Triamine ersetzt sein.

Bevorzugt werden als Komponente a) Diole eingesetzt. Brauchbare Diole sind z. B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine sind z. B. Ethylendiamin, Propylendiamin, 1,4-Diaminobutan, 1,5-Diaminopentan und 1,6-Diaminohexan sowie α,ω-Diaminopolyether, die durch Aminierung von Polyalkylenoxiden mit Ammoniak herstellbar sind.

Bei der Komponente b) handelt es sich bevorzugt um ein Polymerisat mit einem zahlenmittleren Molekulargewicht im Bereich von etwa 300 bis 5 000, bevorzugt etwa 400 bis 4 000, insbesondere 500 bis 3 000. Brauchbare Polymerisate b) sind z. B. Polyesterdiole, Polyetherole und Mischungen davon. Polyetherole sind vorzugsweise Polyalkylenglykole, z. B. Polyethylenglykole, Polypropylenglykole, Polytetrahydrofurane etc., Blockcopolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Vorzugsweise werden als Komponente b) Polytetrahydrofurane, Polyesterdiole und Mischungen davon eingesetzt.

Geeignete Polytetrahydrofurane b) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Brauchbare Polyesterdiole b) weisen vorzugsweise ein zahlenmittleres Molekulargewicht im Bereich von etwa 400 bis 5 000, bevorzugt 500 bis 3 000, insbesondere 600 bis 2 000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan, sowie Poly(meth)acrylatdiole der Formel 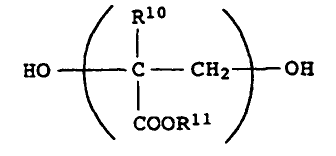 worin R¹⁰ für H oder CH₃ steht und R¹¹ für C₁-C₁₈-Alkyl (insbesondere C₁-C₁₂- oder C₁-C₈-Alkyl) steht, die eine Molmasse von bis zu etwa 3000 aufweisen. Derartige Diole sind auf übliche Weise herstellbar und im Handel erhältlich (Tegomer®-Typen MD, BD und OD der Fa. Goldschmidt).

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% und bevorzugt 50 bis 85 Mol-%, des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/ Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan.

Geeignete Verbindungen c), die zwei aktive Wasserstoffatome und mindestens eine ionogene oder ionische Gruppe pro Molekül aufweisen, sind z. B. Verbindungen mit Carboxylat- und/oder Sulfonatgruppen. Als Komponente c) sind Dimethylolpropansäure und Mischungen, die Dimethylolpropansäure enthalten, besonders bevorzugt.

Geeignete Diamine und/oder Diole c) mit ionogenen oder ionischen Gruppen sind z. B. Dimethylolpropansäure und Verbindungen der Formel 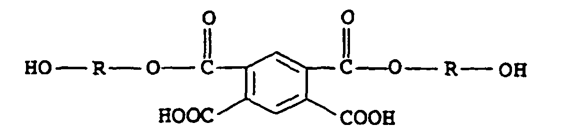 und/oder 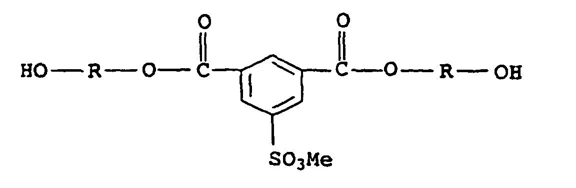 worin R jeweils für eine C₂-C₁₈-Alkylengruppe steht und Me für Na oder K steht.

Als Komponente c) brauchbar sind auch Verbindungen der Formel

H₂N(CH₂)ₙ-NH-(CH₂)ₘ-COO⁻M⁺

H₂N(CH₂)ₙ-NH-(CH₂)ₘ-SO₃⁻ M⁺

worin m und n unabhängig voneinander für eine ganze Zahl von 1 bis 8, insbesondere 1 bis 6, stehen und M für Li, Na oder K steht, und Verbindungen der Formel

H₂N(CH₂CH₂O)ₚ(CH₂CH(CH₃)O)_{q}(CH₂)ₙ-NH-(CH₂)ₘ-SO₃⁻M⁺

worin m, n und M die zuvor angegebenen Bedeutungen besitzen, p und q unabhängig voneinander für eine ganze Zahl von 0 bis 50 stehen, wobei wenigstens eine der beiden Variablen p oder q > 0 ist. Die Reihenfolge der Alkylenoxideinheiten ist dabei beliebig. Die zuletzt genannten Verbindungen weisen vorzugsweise ein zahlenmittleres Molekulargewicht im Bereich von etwa 400 bis 3 000 auf.

Wenn man als Komponente c) Verbindungen mit stickstoffhaltigen Gruppen einsetzt, erhält man kationische Polyurethane. Brauchbare Komponenten c) sind z. B. Verbindungen der allgemeinen Formeln 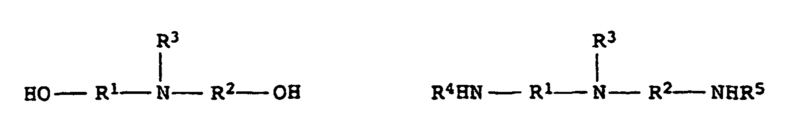 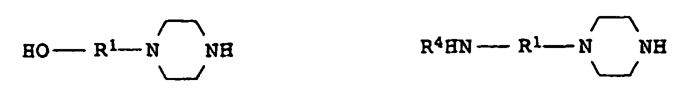 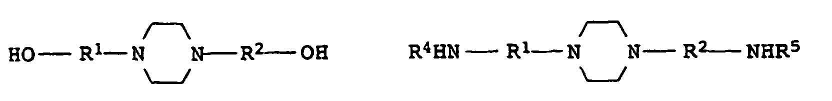 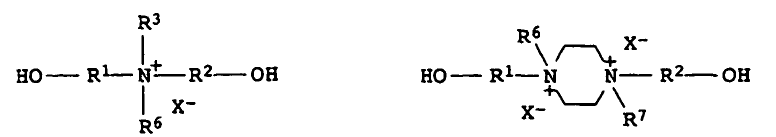 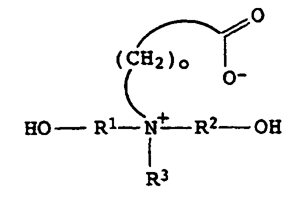 worin
- R¹ und R², die gleich oder verschieden sein können, für C₂-C₈-Alkylen stehen,
- R³, R⁶ und R⁷, die gleich oder verschieden sein können, für C₁-C₆-Alkyl, Phenyl oder Phenyl-C₁-C₄-alkyl stehen,
   R⁴ und R⁵, die gleich oder verschieden sein können, für H oder C₁-C₆-Alkyl stehen,
   o für 1, 2 oder 3 steht,
   X⁻ für Chlorid, Bromid, Jodid, C₁-C₆-Alkylsulfat oder SO₄²⁻/₂ steht. Besonders bevorzugt sind N-(C₁- bis C₆-alkyl)diethanolamine, wie Methyldiethanolamin.

Als Komponente c) eignen sich auch Gemische, die mindestens eine der zuvor genannten anionischen oder anionogenen Komponenten und mindestens eine der zuvor genannten kationischen oder kationogenen Komponenten enthalten. Bevorzugt werden dann Gemische eingesetzt, die Dimethylolpropansäure und N-Methyldiethanolamin enthalten.

Bei der Komponente d) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Tolylendiisocyanat und deren Isomerengemische, o- und m-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat und/oder Dicyclohexylmethandiisocyanat. Gewünschtenfalls können bis zu 3 Mol-% der genannten Verbindungen durch Triisocyanate ersetzt sein.

Die Herstellung der Polyurethanpräpolymere A) erfolgt durch Umsetzung der Verbindungen der Komponenten a), b) und gegebenenfalls c) mit der Komponente d). Die Temperatur liegt dabei in einem Bereich von etwa 60 bis 140 °C, bevorzugt etwa 70 bis 100 °C. Die Reaktion kann ohne Lösungsmittel oder in einem geeigneten inerten Lösungsmittel oder Lösungsmittelgemisch erfolgen. Geeignete Lösungsmittel sind aprotische polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Die Komponenten werden in solchen Mengen eingesetzt, dass das Verhältnis von NCO-Äquivalent der Verbindungen der Komponente d) zu Äquivalent aktives Wasserstoffatom der Komponenten a), b) und gegebenenfalls c) in einem Bereich von etwa 1,0:1 bis 1,4:1, bevorzugt 1,03:1 bis 1,3:1, insbesondere 1,05:1 bis 1,25:1, liegt. Die resultierenden Polyurethanpräpolymere A) weisen somit noch freie Isocyanatgruppen auf.

Vorzugsweise enthalten die Polyurethanpräpolymere
- 0,3 bis 15 Gew.-%, bevorzugt 0,5 bis 12 Gew.-%, wenigstens einer Komponente a),
- 0,5 bis 80 Gew.-%, bevorzugt 1 bis 65 Gew.-%, wenigstens einer Komponente b),
- 5 bis 25 Gew.-%, bevorzugt 8 bis 20 Gew.-%, wenigstens einer Komponente c),
- 25 bis 60 Gew.-%, bevorzugt 35 bis 53 Gew.-%, wenigstens einer Komponente d)
   einpolymerisiert.

Die Herstellung der erfindungsgemäßen, vernetzten, wasserlöslichen oder wasserdispergierbaren Polyurethane erfolgt durch Umsetzung mindestens eines Polyurethanpräpolymers A), wie zuvor beschrieben, mit mindestens einem Polymerisat B). Die Polymerisate B) enthalten:
e) wenigstens ein α,β-ethylenisch ungesättigtes Monomer, das zusätzlich wenigstens eine gegenüber Isocyanatgruppen reaktive Gruppe pro Molekül enthält,
f) gegebenenfalls wenigstens ein α,β-ethylenisch ungesättigtes Monomer, das ausgewählt ist unter Estern α,β-ethylenisch ungesättigter Mono- und/oder Dicarbonsäuren mit C₁- bis C₂₂-Alkanolen, Amiden α,β-ethylenisch ungesättigter Mono- und/oder Dicarbonsäuren mit Mono- und Di-C₁- bis C₂₂-alkylaminen, Estern von Vinylalkohol und Allylalkohol mit C₁- bis C₄₀-Monocarbonsäuren, Vinylethern, Vinylaromaten, Vinylhalogeniden, Vinylidenhalogeniden C₂- bis C₈-Monoolefinen, nichtaromatischen Kohlenwasserstoffen mit mindestens 2 konjugierten Doppelbindungen und Mischungen davon,
g) gegebenenfalls wenigstens ein α,β-ethylenisch ungesättigtes Monomer, das ausgewählt ist unter N-Vinylamiden, N-Vinyllactamen, primären Amiden α,β-ethylenisch ungesättigter Monocarbonsäuren, vinyl- und allylsubstituierten heteroaromatischen Verbindungen und Mischungen davon,
h) gegebenenfalls wenigstens ein weiteres Monomer mit einer radikalisch polymerisierbaren, α,β-ethylenisch ungesättigten Doppelbindung und mindestens einer ionogenen bzw. ionischen Gruppe pro Molekül
einpolymerisiert.

Geeignete Monomere e) sind die Ester α,β-ethylenisch ungesättigter Mono- und Dicarbonsäuren, wie Acrylsäure, Methacrylsäure, Fumarsäure, Maleinsäure, Itaconsäure, Crotonsäure etc., mit C₁-C₂₀-Alkandiolen. Dazu zählen z. B. 2-Hydroxyethylacrylat, 2-Hydroxyethylmethacrylat, 2-Hydroxyethylethacrylat, 2-Hydroxypropylacrylat, 2-Hydroxypropylmethacrylat, 3-Hydroxypropylacrylat, 3-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmethacrylat etc. vorzugsweise werden Hydroxyethylacrylat und Hydroxyethylmethacrylat eingesetzt. Geeignete Monomere e) sind auch die Ester der zuvor genannten Säuren mit Triolen und Polyolen, wie z. B. Glycerin, Erythrit, Pentaerythrit, Sorbit etc.

Geeignete Monomere e) sind weiterhin die Ester und Amide der zuvor genannten α,β-ethylenisch ungesättigten Mono- und Dicarbonsäuren mit C₂- bis C₁₂-Aminoalkoholen, die eine primäre oder sekundäre Aminogruppe aufweisen. Dazu zählen Aminoalkylacrylate und Aminoalkylmethacrylate und deren N-Monoalkylderivate, die z. B. einen N-C₁- bis C₈-Monoalkylrest tragen, wie Aminomethylacrylat, Aminomethylmethacrylat, Aminoethylacrylat, Aminoethylmethacrylat, N-Methylaminomethylacrylat, N-Methylaminomethylmethacrylat, N-Ethylaminomethylacrylat, N-Ethylaminomethylmethacrylat, N-(n-propyl)aminomethyl(meth)acrylat, N-Isopropylaminomethyl-(meth)acrylat und bevorzugt tert.-Butylaminoethylacrylat und tert.-Butylaminoethylmethacrylat. Dazu zählen weiterhin N-(Hydroxy-C₁- bis C₁₂-alkyl)(meth)acrylamide, wie N-Hydroxymethyl(meth)-acrylamid, N-Hydroxyethyl(meth)acrylamid etc.

Geeignete Monomere e) sind auch die Amide der zuvor genannten α,β-ethylenisch ungesättigten Mono- und Dicarbonsäuren mit Di- und Polyaminen, die mindestens zwei primäre oder zwei sekundäre oder eine primäre und eine sekundäre Aminogruppe(n) aufweisen. Dazu zählen z. B. die entsprechenden Amide der Acrylsäure und Methacrylsäure (im Folgenden durch die Silbe "(meth)" bezeichnet), wie Aminomethyl(meth)acrylamid, Aminoethyl(meth)acrylamid, Aminopropyl(meth)acrylamid, Amino-n-butyl(meth)acrylamid, Methylaminoethyl(meth)acrylamid, Ethylaminoethyl(meth)acrylamid, Methylaminopropyl(meth)acrylamid, Ethylaminopropyl(meth)acrylamid, Methylamino-n-butyl(meth)acrylamid etc.

Geeignete Monomere f) sind im Wesentlichen hydrophobe, nichtionische Monomere. Dazu zählen die Ester α,β-ethylenisch ungesättigter Mono- und Dicarbonsäuren mit C₁-C₂₂-Alkanolen, bevorzugt C₁-C₁₈-Alkanolen, z. B. die Ester der Acrylsäure und/oder Methacrylsäure mit Methanol, Ethanol, n-Propanol, Isopropanol, n-Butanol, sec.-Butanol, tert.-Butanol, n-Pentanol, n-Hexanol, n-Heptanol, n-Octanol, 2-Ethylhexanol, Dodecanol, Hexadecanol, Octadecanol etc.

Geeignete Monomere f) sind weiterhin Amide α,β-ethylenisch ungesättigter Mono- und Dicarbonsäuren mit Mono- und Dialkylaminen, die 1 bis 22 Kohlenstoffatome, bevorzugt 1 bis 18 Kohlenstoffatome, pro Alkylrest aufweisen. Dazu zählen z. B. N-C₁- bis C₂₂-Alkyl(meth)acrylamide, wie N-Methyl(meth)acrylamid, N-Ethyl(meth)-acrylamid, N-(n-Propyl)(meth)acrylamid, N-Isopropyl(meth)acrylamid, N-Butyl(meth)acrylamid, N-(t.-Butyl)(meth)acrylamid, N-Pentyl(meth)acrylamid, N-Hexyl(meth)acrylamid, N-Heptyl(meth)-acrylamid, N-Octyl(meth)acrylamid, N-Ethylhexyl(meth)acrylamid, N,N-Dimethyl(meth)acrylamid, N,N-Diethyl(meth)acrylamid etc.

Geeignete Monomere f) sind weiterhin Vinylformiat, Vinylacetat, Vinylpropionat, Vinyl-n-butyrat, Vinylstearat, Vinyllaurat, Styrol, α-Methylstyrol, o-Chlorstyrol, Vinyltoluole, Vinylchlorid, Vinylidenchlorid, Ethylen, Propylen, Butadien, Isopren, Chloropren, Methyl-, Ethyl-, Butyl-, Dodecylvinylether etc.

Geeignete Monomere g) sind im Wesentlichen hydrophile, nichtionische Monomere. Dazu zählen z. B. N-Vinylamide, wie N-Vinylformamid, N-Vinylacetamid, N-Vinylpropionamid etc. Bevorzugt wird N-Vinylformamid eingesetzt.

Geeignete Monomere g) sind weiterhin N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Geeignete Monomere g) sind weiterhin primäre Amide der zuvor genannten α,β-ethylenisch ungesättigten Monocarbonsäuren, wie Acrylamid, Methacrylamid, Ethacrylamid etc.

Geeignete Monomere g) sind weiterhin vinyl- und allylsubstituierte heteroaromatische Verbindungen, wie 2- und 4-Vinylpyridin, -Allylpyridin, und bevorzugt N-Vinylheteroaromaten, wie N-Vinylimidazol, N-Vinyl-2-methylimidazol etc.

Die Verbindungen h) weisen mindestens eine ionogene bzw. ionische Gruppe pro Molekül auf, die bevorzugt ausgewählt ist unter Carboxylatgruppen und/oder Sulfonatgruppen und deren durch teilweise oder vollständige Neutralisation mit einer Base erhältlichen Salze, sowie tertiäre Amingruppen, die teilweise oder vollständig protoniert und quaternisiert sein können. Geeignete Basen für die Neutralisation bzw. Säuren für die Protonierung und Alkylierungsmittel für die Quaternisierung sind die im Folgenden im Anschluss an die Herstellung der erfindungsgemäßen Polyurethane genannten.

Geeignete Monomere h) sind z. B. die zuvor genannten, α,β-ethylenisch ungesättigten Mono- und/oder Dicarbonsäuren und deren Halbester und Anhydride, wie Acrylsäure, Methacrylsäure, Fumarsäure, Maleinsäure, Itaconsäure, Crotonsäure, Maleinsäureanhydrid, Monobutylmaleat etc. Bevorzugt werden Acrylsäure, Methacrylsäure und deren Alkalimetallsalze, wie deren Natrium- und Kaliumsalze, eingesetzt.

Geeignete Monomere h) sind weiterhin Acrylamidoalkansulfonsäuren und deren Salze, wie 2-Acrylamido-2-methylpropansulfonsäure und deren Alkalimetallsalze, z. B. deren Natrium- und Kaliumsalze.

Weitere geeignete Verbindungen h) sind die Ester der zuvor genannten, α,β-ethylenisch ungesättigten Mono- und Dicarbonsäuren mit C₂- bis C₁₂-Aminoalkoholen, welche am Aminstickstoff C₁- bis C₈-dialkyliert sind. Dazu zählen z. B. N,N-Dimethylaminomethyl-(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat etc. Bevorzugt werden N,N-Dimethylaminopropylacrylat und N,N-Dimethylaminopropyl(meth)acrylat eingesetzt.

Geeignete Monomere h) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z. B. N-[2-(dimethylamino)ethyl]-acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)-butyl]methacrylamid, N-[2-(diethylamino)ethyl]acrylamid etc.

Vorzugsweise enthält das Polymerisat B) zusätzlich zur Komponente e) wenigstens eine Komponente f) und/oder g) sowie gegebenenfalls eine Komponente h) einpolymerisiert.

Bevorzugt enthält das Polymerisat B)
- 0,05 bis 15 Gew.-%, bevorzugt 0,1 bis 10 Gew.-%, wenigstens einer Komponente e),
- 0 bis 99,9 Gew.-% wenigstens einer Komponente f),
- 0 bis 99,9 Gew.-% wenigstens einer Komponente g),
- 0 bis 50 Gew.-%, bevorzugt 0,1 bis 46 Gew.-%, wenigstens einer Komponente h),
einpolymerisiert.

Vorzugsweise liegt die Gesamtmenge der Komponenten f) und g) in einem Bereich von 30 bis 99,9 Gew.-%, insbesondere 40 bis 99,5 Gew.-%, speziell 50 bis 99,5 Gew.-%.

Nach einer bevorzugten Ausführungsform enthält das Polymerisat B)
- 0,1 bis 10 Gew.-%, bevorzugt 0,5 bis 5 Gew.-%, wenigstens einer Komponente e)
- 50 bis 99,9 Gew.-%, bevorzugt 60 bis 99,5 Gew.-%, wenigstens einer Komponente g),
- 0 bis 40 Gew.-%, bevorzugt 0 bis 35 Gew.-%, wenigstens einer Komponente h),
einpolymerisiert.

Nach einer weiteren bevorzugten Ausführungsform enthält das Polymerisat B)
- 0,1 bis 10 Gew.-%, bevorzugt 0,5 bis 5 Gew.-%, wenigstens einer Komponente e),
- 50 bis 99,9 Gew.-%, bevorzugt 60 bis 90 Gew.-%, wenigstens einer Komponente f),
- 0 bis 50 Gew.-%, bevorzugt 10 bis 46 Gew.-%, wenigstens einer Komponente h),
einpolymerisiert.

Nach einer weiteren bevorzugten Ausführungsform enthält das Polymerisat B)
- 0,1 bis 10 Gew.-%, bevorzugt 0,5 bis 5 Gew.-%, wenigstens einer Komponente e),
- 0,1 bis 99,9 Gew.-%, bevorzugt 0,1 bis 90 Gew.-%, wenigstens einer Komponente f),
- 0,1 bis 99,9 Gew.-%, bevorzugt 0,1 bis 99,5 Gew.-%, wenigstens einer Komponente g),
- 0 bis 50 Gew.-%, bevorzugt 0 bis 46 Gew.-%, wenigstens einer Komponente h),
einpolymerisiert.

Ein weiterer Gegenstand der Erfindung ist ein Polymerisat B), das
- 0,05 bis 15 Gew.-%, bevorzugt 0,1 bis 10 Gew.-% einer Komponente e), wie in Anspruch 5 definiert, vorzugsweise tert.-Butylaminoethylacrylat und/oder tert.-Butylaminoethylmethacrylat,
- 0 bis 99,9 Gew.-% wenigstens einer Komponente f),
- 0,1 bis 99,9 Gew.-% wenigstens einer Komponente g),
- 0 bis 50 Gew.-%, bevorzugt 0,1 bis 46 Gew.-%, wenigstens einer Komponente h)
einpolymerisiert enthält. Die Gesamtmenge der Komponenten f) und g) liegt vorzugsweise in einem Bereich von 30 bis 99,9 Gew.-%.

Die Herstellung der Polymerisate B) erfolgt nach üblichen, dem Fachmann bekannten Verfahren. Dazu zählt die Polymerisation in Masse und vorzugsweise die Lösungspolymerisation. Die Polymerisationstemperatur beträgt in der Regel 30 bis 120 °C, bevorzugt 40 bis 100 °C. Das Polymerisationsmedium kann sowohl nur aus einem organischen Lösungsmittel als auch aus Mischungen aus Wasser und mindestens einem wassermischbaren, organischen Lösungsmittel bestehen. Bevorzugte organische Lösungsmittel sind z. B. Alkohole, wie Methanol, Ethanol, n-Propanol, Isopropanol, n-Butanol, Ketone, wie Aceton und Methylethylketon, Tetrahydrofuran etc. Die Lösungspolymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Als Initiatoren für die radikalische Polymerisation kommen Azoverbindungen in Frage, die für die radikalische Polymerisation geeignet sind. Dazu zählen aliphatische oder cycloaliphatische Azoverbindungen, z. B. 2,2'-Azobis(isobutyronitril), 2,2'-Azo-bis(2-methylbutyronitril), 2,2'-Azobis(2,4-dimethylvaleronitril), 1,1'-Azobis(1-cyclohexancarbonitril), 2-(Carbamoylazo)isobutyronitril, 4,4'-Azobis(4-cyanovaleriansäure) und deren Alkalimetall- und Ammoniumsalze, z. B. das Natriumsalz, Dimethyl-2,2'-azobisisobutyrat, 2,2'-Azobis[2-(2-imidazolin-2-yl)propan], 2,2'-Azo-bis(2-amidinopropan) und die Säureadditionssalze der beiden zuletzt genannten Verbindungen, z. B. die Dihydrochloride.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Allgemeinen in einem Bereich von etwa 0,02 bis 15 Mol-%, vorzugsweise 0,05 bis 3 Mol-%.

Sind niedrigere Molekulargewichte erwünscht, so können diese durch Zusatz eines Reglers zum Polymerisationsansatz eingestellt werden. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Gewünschtenfalls setzt man der Polymerlösung im Anschluss an die Polymerisationsreaktion einen oder mehrere Polymerisationsinitiatoren zu und erhitzt die Polymerlösung, z. B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z. B. von 99,9 %, geführt. Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z. B. Destillation bei verringertem Druck, teilweise oder vollständig entfernt und gegebenenfalls gegen das für die folgende Umsetzung des Polymerisats B) mit dem Polyurethanpräpolymer A) eingesetzte Lösungsmittel ausgetauscht werden. Dabei werden vorzugsweise hydroxylgruppenhaltige Polymerisate B), die in einem Lösungsmittel mit aktiven Wasserstoffatomen hergestellt wurden, vor der Umsetzung mit A) getrocknet und anschließend in einem Lösungsmittel oder Lösungsmittelgemisch eingesetzt, das keine aktiven Wasserstoffatome aufweist.

Die Herstellung der erfindungsgemäßen Polyurethane erfolgt durch Umsetzung des Polyurethanpräpolymers A) mit dem Polymerisat B). Dabei liegt das Verhältnis von NCO-Äquivalent der Komponente A) zu Äquivalent aktives Wasserstoffatom der Komponente B) im Allgemeinen in einem Bereich von etwa 20:1 bis 1:1, bevorzugt 10:1 bis 1:1, insbesondere 10:1 bis 1,01:1. Die Temperatur bei der Umsetzung liegt im Allgemeinen in einem Bereich von etwa 10 bis 150 °C, bevorzugt etwa 20 bis 90 °C. Die Reaktion kann vorzugsweise in einem geeigneten inerten Lösungsmittel oder Lösungsmittelgemisch erfolgen. Geeignete Lösungsmittel sind die zuvor für die Herstellung der Polyurethanpräpolymere A) genannten. Wird als Komponente B) ein hydroxylgruppenhaltiges Polymerisat eingesetzt, so liegt die Reaktionstemperatur bevorzugt in einem Bereich von etwa 60 bis 150 °C. Die Reaktion erfolgt dann vorzugsweise in einem Lösungsmittel oder Lösungsmittelgemisch, welches keine aktiven Wasserstoffatome aufweist. Bevorzugt werden Ketone, wie Aceton, Methylethylketon und Gemische davon eingesetzt. Wird als Komponente B) ein Polymerisat eingesetzt, das als gegenüber Isocyanatgruppen reaktive Gruppen überwiegend oder ausschließlich primäre und/oder sekundäre Aminogruppen aufweist, so liegt die Reaktionstemperatur bevorzugt in einem Bereich von etwa 20 bis 80 °C. Die Reaktion kann dann gewünschtenfalls in einem Lösungsmittel oder Lösungsmittelgemisch erfolgen, welches aktive Wasserstoffatome aufweisen kann. Neben den zuvor Genannten werden bevorzugt Alkohole, wie Methanol und Ethanol, Gemische aus Alkoholen und Wasser sowie Gemische aus Alkoholen und den zuvor genannten Ketonen eingesetzt. Vorzugsweise wird zur Herstellung der erfindungsgemäßen Polyurethane eine Lösung einer der Komponenten A) oder B) in einem üblichen, dem Fachmann bekannten Reaktor, z. B. einem Rührreaktor, vorgelegt. Die zweite Komponente wird dann vorzugsweise ebenfalls in Form einer Lösung zugegeben und die Umsetzung nach Beendigung der Zugabe solange fortgeführt, bis der NCO-Gehalt des Gemisches konstant bleibt. Weisen die resultierenden Polyurethane noch freie Isocyanatgruppen auf, so werden diese abschließend durch Zusatz von Aminen, vorzugsweise Aminoalkoholen, inaktiviert. Geeignete Aminoalkohole sind die zuvor beschriebenen, bevorzugt 2-Amino- 2-methyl-1-propanol.

Die Säuregruppen enthaltenden Polyurethane können mit einer Base teilweise oder vollständig neutralisiert werden.

In aller Regel weisen die erhaltenen Salze der Polyurethane eine bessere Wasserlöslichkeit oder Dispergierbarkeit in Wasser auf als die nicht neutralisierten Polyurethane. Als Base für die Neutralisation der Polyurethane können Alkalimetallbasen wie Natronlauge, Kalilauge, Soda, Natriumhydrogencarbonat, Kaliumcarbonat oder Kaliumhydrogencarbonat und Erdalkalimetallbasen wie Calciumhydroxyd, Calciumoxid, Magnesiumhydroxyd oder Magnesiumcarbonat sowie Ammoniak und Amine verwendet werden. Geeignete Amine sind z. B. C₁-C₆-Alkylamine, bevorzugt n-Propylamin und n-Butylamin, Dialkylamine, bevorzugt Diethylpropylamin und Dipropylmethylamin, Trialkylamine, bevorzugt Triethylamin und Triisopropylamin, C₁-C₆-Alkyldiethanolamine, bevorzugt Methyl- oder Ethyldiethanolamin und Di-C₁-C₆-Alkylethanolamine. Besonders für den Einsatz in Haarbehandlungsmitteln haben sich zur Neutralisation der Säuregruppen enthaltenden Polyurethane 2-Amino-2-methyl-1-propanol, Diethylaminopropylamin und Triisopropanolamin bewährt. Gewünschtenfalls kann zur Neutralisation auch ein siloxangruppenhaltiges Amin, vorzugsweise ein Monoamin, z. B. 3-Aminopropyltrimethoxysilan, eingesetzt werden. Die Neutralisation der Säuregruppen enthaltenden Polyurethane kann auch mit Hilfe von Mischungen mehrerer Basen vorgenommen werden, z. B. Mischungen aus Natronlauge und Triisopropanolamin. Die Neutralisation kann je nach Anwendungszweck partiell z. B. zu 20 bis 40 % oder vollständig, d. h. zu 100 % erfolgen.

Die Amingruppen bzw. protonierte oder quaternisierte Amingruppen enthaltenden Polyurethane sind aufgrund ihrer kationischen Gruppen im Allgemeinen leicht in Wasser oder Wasser/Alkohol-Gemischen löslich oder zumindest ohne Zuhilfenahme von Emulgatoren dispergierbar. Geladene kationische Gruppen lassen sich aus den vorliegenden tertiären Aminstickstoffen entweder durch Protonierung, z. B. mit Carbonsäuren, wie Milchsäure, oder Mineralsäuren, wie Phosphorsäure, Schwefelsäure und Salzsäure, oder durch Quaternisierung, z. B. mit Alkylierungsmitteln, wie C₁- bis C₄-Alkylhalogeniden oder -sulfaten, erzeugen. Beispiele solcher Alkylierungsmittel sind Ethylchlorid, Ethylbromid, Methylchlorid, Methylbromid, Dimethylsulfat und Diethylsulfat.

Nach einer geeigneten Ausführungsform können die erfindungsgemäßen Polyurethane sowohl Säuregruppen als auch Aminogruppen aufweisen. Der Betrag der Differenz aus Säuregruppen und Aminogruppen (|ΔSZ-AZ|) liegt dabei vorzugsweise in einem Bereich von etwa 15 bis 150, bevorzugt 30 bis 100. Säurezahl und Aminzahl sind dabei jeweils als mg KOH/g Prüfsubstanz definiert.

Wird bei der Herstellung der Polyurethane ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem Polyurethan zusätzlich Wasser zugegeben werden. Nach Ersatz des Lösungsmittels durch Wasser erhält man eine Lösung oder Dispersion des Polymers, aus der, falls gewünscht, das Polymer in üblicher Weise gewonnen werden kann, z. B. durch Sprühtrocknung.

Die erfindungsgemäßen Polyurethane weisen keine Siloxangruppen auf. Ihre K-Werte (gemessen nach E. Fikentscher, Cellulose-Chemie 13 (1932), S. 58-64, an einer 1%igen Lösung in N-Methylpyrrolidon) liegen im Allgemeinen in einem Bereich von etwa 15 bis 90, bevorzugt 20 bis 60. Ihre Glasübergangstemperatur beträgt im Allgemeinen mindestens 0 °C, bevorzugt mindestens 20 °C, insbesondere bevorzugt mindestens 25 °C und speziell mindestens 30 °C. Weisen die erfindungsgemäßen Polyurethane zwei oder mehrere Glasübergangstemperaturen auf, so liegt wenigstens eine davon in dem angegebenen Bereich. Bevorzugt liegt/liegen die andere(n) dann unterhalb des zuvor angegebenen Temperaturbereichs.

Die erfindungsgemäßen Polyurethane sind als Hilfsmittel in der Kosmetik und Pharmazie, insbesondere als oder in Beschichtungsmittel(n) für keratinhaltige Oberflächen (Haar, Haut und Nägel) und als Überzugsmittel und/oder Bindemittel für feste Arzneiformen brauchbar. Außerdem sind sie als oder in Beschichtungsmittel(n) für die Textil-, Papier-, Druck-, Leder- und Klebstoffindustrie brauchbar. Sie sind insbesondere in der Haarkosmetik brauchbar. Die zuvor genannten Polyurethane können auch in Cremes und als Tablettenüberzugmittel und Tablettenbindemittel verwendet werden. Sie eignen sich auch als Bindemittel und Klebemittel für kosmetische Produkte, z. B. bei der Herstellung stiftförmiger kosmetischer Produkte, wie Deostifte, Schminkstifte etc.

Gegenstand der vorliegenden Erfindung ist auch ein kosmetisches oder pharmazeutisches Mittel, das die erfindungsgemäßen Polyurethane enthält. Im Allgemeinen enthält das Mittel die Polyurethane in einer Menge im Bereich von 0,2 bis 30 Gew.-%, bezogen auf das Gesamtgewicht des Mittels.

Die erfindungsgemäßen kosmetischen Mittel eignen sich insbesondere als Beschichtungsmittel für keratinhaltige Oberflächen (Haar, Haut und Nägel). Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise 1 bis 250 nm, bevorzugt 1 bis 150 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, insbesondere in Form eines Haarsprays vorliegen. Zur Anwendung als Haarfestiger sind dabei Mittel bevorzugt, die Polyurethane enthalten, die wenigstens eine Glasübergangstemperatur T_{g} ≥ 20 °C, bevorzugt ≥ 30 °C, aufweisen. Der K-Wert dieser Polymere liegt vorzugsweise in einem Bereich von 23 bis 90, insbesondere 25 bis 60.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter enthalten die erfindungsgemäßen Haarbehandlungsmittel im Allgemeinen übliche kosmetische Hilfsstoffe, beispielsweise Weichmacher, wie Glycerin und Glykol; Emollienzien; Parfüms; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen;
- amphotere oder zwitterionische Polymere, wie die unter den Bezeichnungen Amphomer® (Delft National) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacryloylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer, Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus vinylpyrrolidon und vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen vernetzten Polyurethane können als Mischung mit einem anderen amidgruppenhaltigen Haarpolymer eingesetzt. Dazu zählen z. B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z. B. Ultrahold®strong der BASF AG), die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die erfindungsgemäßen vernetzten Polyurethane können auch als Mischung mit einem anderen, siloxangruppenhaltigen Haarpolymer eingesetzt werden, vorzugsweise siloxangruppenhaltigen Polyurethanen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-% mindestens eines, in Wasser löslichen oder dispergierbaren, erfindungsgemäßen Polyurethans,
b) 40 bis 99 Gew.-%, bevorzugt 50 bis 98 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 50 Gew.-% eines Treibmittels, vorzugsweise Dimethylether,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers,
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 15 Gew.-%, bevorzugt 0,1 bis 10 Gew.-%, bezogen auf das Gesamtgewicht des Mittels. Bevorzugt können dabei wasserlösliche oder wasserdispergierbare Polyurethane eingesetzt werden, die Siloxangruppen einpolymerisiert enthalten.

Das erfindungsgemäße Mittel kann als Komponente e) mindestens ein wasserunlösliches Silicon, insbesondere ein Polydimethylsiloxan, z. B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann als Komponente f) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Die erfindungsgemäßen Mittel besitzen den Vorteil, dass sie einerseits den Haaren die gewünschte Festigkeit verleihen und andererseits die Polymere leicht auswaschbar (redispergierbar) sind. Darüber hinaus lassen sich Haarbehandlungsmittel mit einem in VOC-Gehalt von weniger als 85 Gew.-%, bevorzugt weniger als 60 Gew.-%, und auch rein wässrige Formulierungen herstellen, selbst wenn sie als Haarspray formuliert sind.

Die Erfindung wird anhand der folgenden, nicht einschränkenden Beispiele näher erläutert.

### Beispiele

### Beispiele 1 bis 4

### Polyurethanpräpolymerherstellung

In einem Rührapparat, der mit Rührer, Tropftrichter, Thermometer, Rückflusskühler und einer Vorrichtung für das Arbeiten unter Stickstoff ausgestattet war, wurden ein Polyesterdiol (Mₙ = 1 000 g/mol, hergestellt aus Isophthalsäure, Adipinsäure und Hexandiol) (Beispiele 1 bis 3) und/oder ein Polytetrahydrofuran (Mₙ = 1 000 g/mol) (Beispiele 3, 4), Cyclohexandimethylol (Beispiel 1) oder Neopentylglykol (Beispiele 2 bis 4), Dimethylolpropansäure und gegebenenfalls Methyldiethanolamin (Beispiel 4) in einer Menge nach Tabelle 1 in Methylethylketon (Feststoffgehalt der resultierenden Reaktionslösung ca. 75 %) unter Erhitzen auf eine Temperatur von etwa 70 °C und unter Rühren gelöst. Anschließend wurde unter Rühren Isophorondiisocyanat in einer Menge nach Tabelle 1 zugetropft, wobei die Reaktionstemperatur anstieg. Bei einer Innentemperatur von 85 °C wurde das Reaktionsgemisch dann solange gerührt, bis der Isocyanatgruppengehalt des Gemisches praktisch konstant blieb (ca. 0,5 bis 1 %). Das Reaktionsgemisch wurde mit Methylethylketon auf 40 Gew.-% verdünnt und unter Rühren auf Raumtemperatur abgekühlt.

**Tabelle 1**

| Bsp. Nr. | Polyesterdiol¹⁾ [mol] | Poly(THF)²⁾ [mol] | CHDM³⁾ [mol] | NPG⁴⁾ [mol] | DMPA⁵⁾ [mol] | MDEA⁶⁾ [mol] | IPDI⁷⁾ [mol] |
|---|---|---|---|---|---|---|---|
| 1 | 0,8 | - | 1,7 | - | 3 | - | 6 |
| 2 | 1,2 | - | - | 1,4 | 3 | - | 6 |
| 3 | 0,7 | 0,7 | - | 1,2 | 3 | - | 6 |
| 4 | - | 1 | - | 1,0 | 3 | 0,5 | 6 |

| | | | | | | | |
|---|---|---|---|---|---|---|---|
| ¹⁾ Polyesterdiol aus Isophthalsäure, Adipinsäure, Hexandiol, Mₙ = 1 000 g/mol | | | | | | | |
| ²⁾ Polytetrahydrofuran, Mₙ = 1 000 g/mol | | | | | | | |
| ³⁾ CHDM = Cyclohexandimethylol | | | | | | | |
| ⁴⁾ NPG = Neopentylglykol | | | | | | | |
| ⁵⁾ DMPA = Dimethylolpropansäure | | | | | | | |
| ⁶⁾ MDEA = N-Methyldiethanolamin | | | | | | | |
| ⁷⁾ IPDI = Isophorondiisocyanat | | | | | | | |

### Beispiele 5 bis 21

### Herstellung des Polymerisates B)

| | | |
|---|---|---|
| Zulauf 1 | 300 g | Monomerengemisch nach Tabelle 2 |
| | 30 g | Lösungsmittel: |
| | | Beispiele 5, 6, 8-13, 17-21: Ethanol |
| | | Beispiele 7, 14-16: Ethanol/Wasser (1:1) |
| Zulauf 2 | 0,6 g | 2,2'-Azobis(2-methylbutyronitril) |
| | 150 g | Ethanol |
| Zulauf 3 | 3,0 g | 2,2'-Azobis(2-methylbutyronitril) |
| | 150 g | Ethanol |

In einer Rührapparatur mit Rückflusskühler und zwei separaten Zulaufvorrichtungen wurden 20 Gew.-% von Zulauf 1 (Monomerengemisch gemäß Tabelle 2), 12 Gew.-% von Zulauf 2 und 120 g Ethanol vorgelegt und die Mischung auf ca. 75 °C aufgeheizt. Nach dem Anpolymerisieren, erkennbar an einer beginnenden Viskositätserhöhung, wurde der Rest von Zulauf 1 innerhalb von 4 Stunden und der Rest von Zulauf 2 innerhalb von 5 Stunden zugegeben, wobei die Innentemperatur auf etwa 70 bis 75 °C gehalten wurde. Anschließend wurde Zulauf 3 innerhalb von 2 Stunden zugegeben, wobei die Innentemperatur auf ca. 80 °C erhöht wurde. Nach dem Ende der Zugabe wurde noch ca. 5 Stunden bei dieser Temperatur nachpolymerisiert. Die so erhaltenen Polymerisate können ohne weitere Maßnahmen zur Verringerung des Restmonomerengehalts zur Polyurethanherstellung eingesetzt werden. Polymerisate mit sehr geringen Restmonomergehalten werden erhalten, wenn man statt Zulauf 3 z. B. 2,5-Bis-(tert.-butylperoxy-2,5-dimethylhexan) (Trigonox® 101 der Fa. Akzo Nobel) in 150 g Ethanol innerhalb von 2 Stunden zu dem Reaktionsgemisch gibt und anschließend noch etwa 5 Stunden bei einer Temperatur von ca. 130 °C unter dem Eigendruck des Reaktionsgemisches nachpolymerisiert.

Die hydroxylgruppenhaltigen Polymerisate der Beispiele 5 bis 8 werden im Anschluss an die Polymerisation durch Sprühtrocknung getrocknet und dann werden für die weitere Umsetzung 40 gew.-%ige Lösungen dieser Polymerisate in Methylethylketon hergestellt.

Bei den in einem Lösungsmittelgemisch aus Ethanol/Wasser (1:1) hergestellten Polymerisaten der Beispiele 14 bis 16 wird das Lösungsmittel durch Destillation unter verringertem Druck bei etwa 40 °C entfernt und dann werden für die weitere Umsetzung 40 gew.-%ige Lösungen in Ethanol hergestellt.

Die ethanolischen Lösungen der übrigen Polymerisate werden durch Zugabe von weiterem Ethanol ebenfalls auf 40 Gew.-% eingestellt.

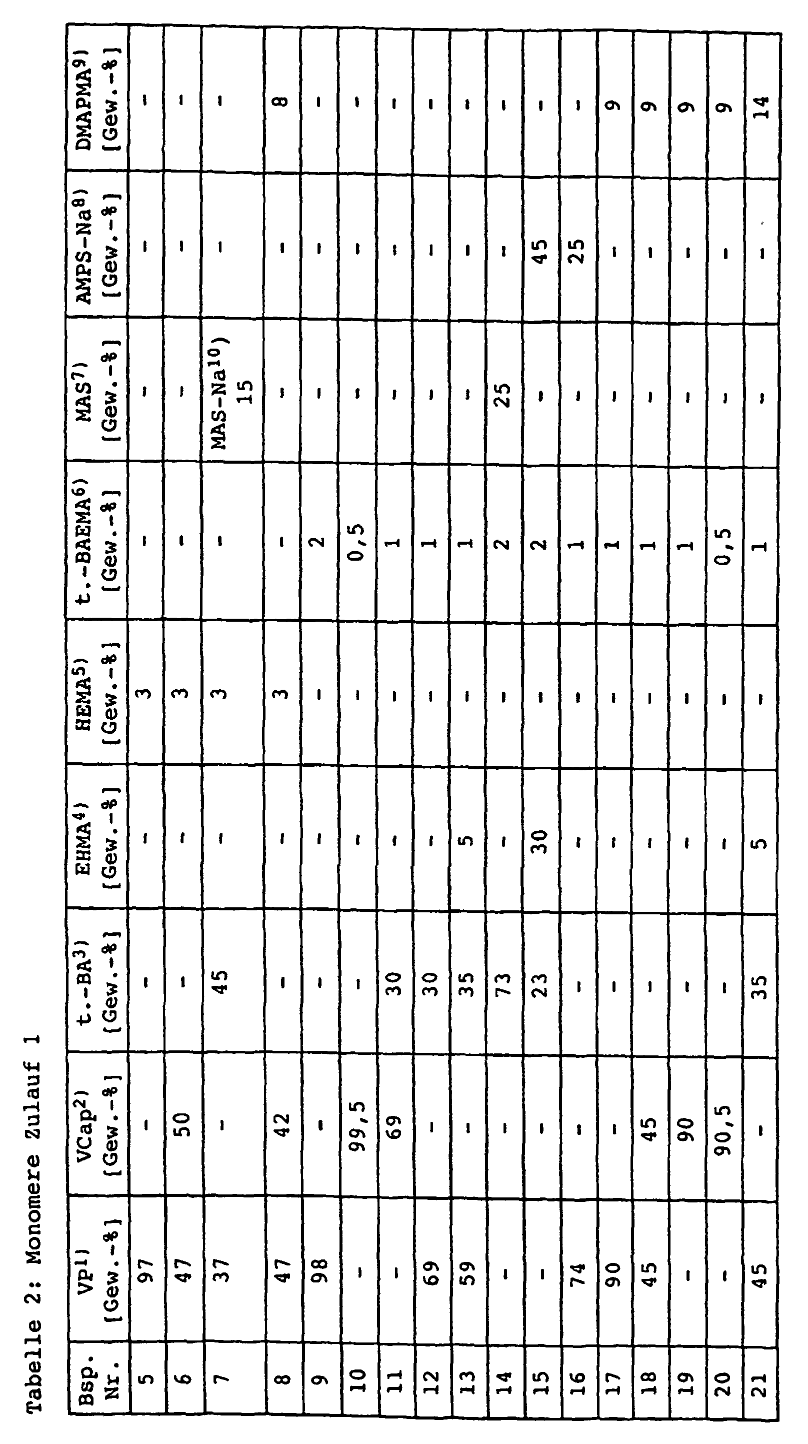

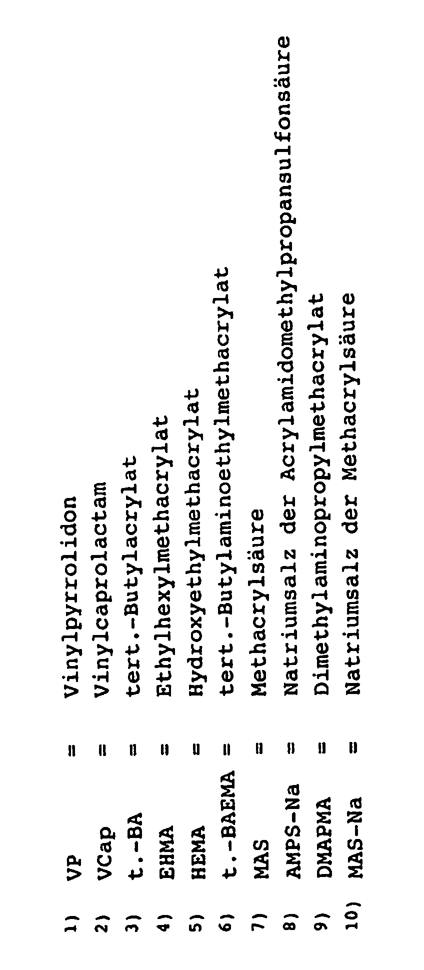

### Beispiele 22 bis 26

### Polyurethanherstellung

In einem Rührapparat, der mit Rührer, Tropftrichter, Thermometer und Rückflusskühler ausgestattet war, wurde eine 40 gew.-%ige Lösung eines Polyurethanpräpolymers aus Beispiel 1 bis 3 in Methylethylketon, wie in Tabelle 3 angegeben, vorgelegt und auf 60 °C erhitzt. Anschließend wurde eine 40 gew.-%ige Lösung eines hydroxylgruppenhaltigen Polymerisates B) aus Beispiel 5 bis 8, ebenfalls in Methylethylketon, nach Tabelle 3 zugemischt. Das Reaktionsgemisch wurde bei einer Temperatur von etwa 85 °C solange gerührt, bis der Isocyanatgruppengehalt des Gemisches praktisch konstant blieb (etwa 2 Stunden). Anschließend gab man Wasser zum Reaktionsgemisch und neutralisierte das Reaktionsprodukt mit 2-Amino-2-methylpropanol (pH etwa 8,0). Das Methylethylketon wurde dann im Vakuum bei 40 °C abdestilliert, wobei eine wässrige Dispersion des Polyurethans erhalten wurde.

Ein pulverförmiges Produkt kann durch Sprühtrocknung erhalten werden.

### Beispiele 27 bis 41

In einem Rührapparat, der mit Rührer, Tropftrichter, Thermometer und Rückflusskühler ausgestattet war, wurde eine 40 gew.-%ige Lösung eines Polyurethanpräpolymers aus Beispiel 1 bis 4 in Methylethylketon gemäß Tabelle 3 vorgelegt. Bei einer Temperatur von etwa 30 °C wurde dann ein Polymerisat B) gemäß Tabelle 3 in Form einer 40 gew.-%igen Lösung in Ethanol zugemischt. Das Reaktionsgemisch wurde dann etwa 1 Stunde bei Umgebungstemperatur gerührt. Anschließend gab man Wasser zum Reaktionsgemisch und neutralisierte das Reaktionsprodukt mit 2-Amino-2-methylpropanol (pH etwa 8,0). Das Methylethylketon wurde dann im Vakuum bei 40 °C abdestilliert, wobei eine wässrige Dispersion des Polyurethans erhalten wurde.

Ein pulverförmiges Produkt kann durch Sprühtrocknung erhalten werden.

**Tabelle 3:**

| Bsp. Nr. | PU-Präpolymer | | Polymerisat B) | | K-Wert¹⁾ |
|---|---|---|---|---|---|
| | Bsp. Nr. | [Gew.-%] | Bsp. Nr. | [Gew.-%] | |
| 22 | 1 | 80 | 5 | 20 | 33 |
| 23 | 1 | 50 | 6 | 50 | 37,3 |
| 24 | 1 | 90 | 8 | 10 | 32,4 |
| 25 | 2 | 90 | 8 | 10 | 34,7 |
| 26 | 3 | 90 | 8 | 10 | 36,1 |
| 27 | 1 | 90 | 9 | 10 | 34,7 |
| 28 | 1 | 70 | 10 | 30 | 35,4 |
| 29 | 1 | 90 | 14 | 10 | 37,6 |
| 30 | 1 | 80 | 16 | 20 | 39,4 |
| 31 | 1 | 80 | 18 | 20 | 31,2 |
| 32 | 2 | 80 | 18 | 20 | 32 |
| 33 | 3 | 80 | 18 | 20 | 35,3 |
| 34 | 1 | 90 | 21 | 10 | 32,1 |
| 35 | 1 | 80 | 21 | 20 | 37,4 |
| 36 | 1 | 20 | 21 | 80 | 45,2 |
| 37 | 3 | 50 | 10 | 50 | 42 |
| 38 | 4 | 50 | 10 | 50 | 38,7 |
| 39 | 4 | 90 | 10 | 10 | 32,1 |
| 40 | 4 | 90 | 18 | 10 | 33,2 |
| 41 | 4 | 10 | 14 | 90 | 42,8 |

| | | | | | |
|---|---|---|---|---|---|
| ¹⁾ 1 gew.-%ige Lösung in N-Methylpyrrolidon | | | | | |

Die zuvor beschriebenen wässrigen Dispersionen der erfindungsgemäßen vernetzten Polyurethane können direkt für die Herstellung von Haarsprayformulierungen eingesetzt werden.

### Anwendungstechnische Beispiele

### Beispiele 42 bis 61

### Aerosol-Haarspray-Formulierungen mit einem VOC-Gehalt von 97 Gew.-%:

| | |
|---|---|
| Polyurethan gemäß Beispiel 22-41 | 3,00 Gew.-% |
| Ethanol | 62,00 Gew.-% |
| Dimethylether | 34,96 Gew.-% |
| Parfüm, Zusatzstoffe | q.s. |

### Beispiele 62 bis 76

### Kompakte Aerosol-Haarspray-Formulierungen mit einem VOC-Gehalt von 90 Gew.-%:

| | |
|---|---|
| Polyurethan gemäß Beispiel 22-36 | 10,00 Gew.-% |
| Ethanol | 55,00 Gew.-% |
| Dimethylether | 34,96 Gew.-% |
| Parfüm, Zusatzstoffe | q.s. |

### Beispiele 77 bis 96

### Haarspray-Formulierungen mit einem VOC-Gehalt von 80 Gew.-%:

| | |
|---|---|
| Polyurethan gemäß Beispiel 22-41 | 5,00 Gew.-% |
| Ethanol | 45,00 Gew.-% |
| Wasser | 15,00 Gew.-% |
| Dimethylether | 34,96 Gew.-% |
| Parfüm, Zusatzstoffe | q.s. |

### Beispiele 97 bis 116

### Haarspray-Formulierungen mit einem VOC-Gehalt von 55 Gew.-%:

| | |
|---|---|
| Polyurethan gemäß Beispiel 22-41 | 5,00 Gew.-% |
| Ethanol | 20,00 Gew.-% |
| Wasser | 40,00 Gew.-% |
| Dimethylether | 34,96 Gew.-% |
| Parfüm, Zusatzstoffe | q.s. |

### Beispiele 117 bis 136

### Pump-Haarspray-Formulierungen mit VOC-Gehalt 0:

| | |
|---|---|
| Polyurethan gemäß Beispiel 22-41 | 10,00 Gew.-% |
| Wasser | 89,97 Gew.-% |
| Parfüm, Zusatzstoffe | q.s. |

## Patentansprüche

1. Kosmetisches oder pharmazeutisches Mittel, enthaltend wenigstens ein vernetztes, wasserlösliches oder wasserdispergierbares Polyurethane aus
A) mindestens einem wasserlöslichen oder wasserdispergierbaren Polyurethanpräpolymer mit endständigen Isocyanatgruppen aus
a) mindestens einer Verbindung mit einem Molekulargewicht im Bereich von 56 bis 300, die zwei aktive Wasserstoffatome pro Molekül enthält,
b) mindestens einem Polymerisat mit zwei aktiven Wasserstoffatomen pro Molekül,
c) mindestens einer Verbindung, die zwei aktive Wasserstoffatome und mindestens eine ionogene bzw. ionische Gruppe pro Molekül aufweist,
d) mindestens einem Diisocyanat,
B) mindestens einem Polymerisat mit gegenüber Isocyanatgruppen reaktiven Gruppen, die ausgewählt sind unter Hydroxyl-, primären und sekundären Amino- und/oder Carboxylgruppen,
oder die Salze davon.

2. Mittel nach Anspruch 1, wobei das Verhältnis von NCO-Äquivalent der Verbindungen der Komponente d) zu Äquivalent aktives Wasserstoffatom der Komponenten a), b) und c) in einem Bereich von 1,01:1 bis 1,4:1, bevorzugt 1,03:1 bis 1,3:1, insbesondere 1,05:1 bis 1,25:1, liegt.

3. Mittel nach einem der Ansprüche 1 oder 2, wobei das Polymerisat B)
e) wenigstens ein α,β-ethylenisch ungesättigtes Monomer, das zusätzlich wenigstens eine gegenüber Isocyanatgruppen reaktive Gruppe pro Molekül enthält,
f) gegebenenfalls wenigstens ein α,β-ethylenisch ungesättigtes Monomer, das ausgewählt ist unter Estern α,β-ethylenisch ungesättigter Mono- und/oder Dicarbonsäuren mit C₁-bis C₂₂-Alkanolen, Amiden α,β-ethylenisch ungesättigter Mono- und/oder Dicarbonsäuren mit Mono- und Di-C₁- bis C₂₂-alkylaminen, Estern von Vinylalkohol und Allylalkohol mit C₁- bis C₄₀-Monocarbonsäuren, Vinylethern, Vinylaromaten, Vinylhalogeniden, Vinylidenhalogeniden, C₂- bis C₈-Monoolefinen, nichtaromatischen Kohlenwasserstoffen mit mindestens 2 konjugierten Doppelbindungen und Mischungen davon,
g) gegebenenfalls wenigstens ein α,β-ethylenisch ungesättigtes Monomer, das ausgewählt ist unter N-Vinylamiden, N-Vinyllactamen, primären Amiden α,β-ethylenisch ungesättigter Monocarbonsäuren, vinyl- und allylsubstituierten heteroaromatischen Verbindungen und Mischungen davon,
h) gegebenenfalls wenigstens ein weiteres Monomer mit einer radikalisch polymerisierbaren, α,β-ethylenisch ungesättigten Doppelbindung und mindestens einer ionogenen bzw. ionischen Gruppe pro Molekül,
einpolymerisiert enthält.

4. Mittel nach Anspruch 3, wobei das Polymerisat B)
- 0,05 bis 15 Gew.-%, bevorzugt 0,1 bis 10 Gew.-%, wenigstens einer Komponente e),
- 0 bis 99,9 Gew.-% wenigstens einer Komponente f),
- 0 bis 99,9 Gew.-% wenigstens einer Komponente g),
- 0 bis 50 Gew.-%, bevorzugt 0,1 bis 46 Gew.-%, wenigstens einer Komponente h)
einpolymerisiert enthält, wobei die Gesamtmenge der Komponenten f) und g) in einem Bereich von 30 bis 99,9 Gew.-% liegt.

5. Mittel nach einem der Ansprüche 3 oder 4, wobei es sich bei der Komponente e) um einen Ester der Acrylsäure oder Methacrylsäure mit einem C₂- bis C₁₂-Aminoalkohol handelt, wobei der Aminstickstoff zusätzlich einen C₁- bis C₈-Alkylrest tragen kann.

6. Mittel nach einem der vorhergehenden Ansprüche, wobei das Verhältnis von NCO-Äquivalent der Komponente A) zu Äquivalent aktives Wasserstoffatom der Komponente B) in einem Bereich von 20:1 bis 1:1, bevorzugt 10:1 bis 1:1, liegt.

7. Mittel nach einem der vorhergehenden Ansprüche, enthaltend
a) 0,5 bis 20 Gew.-% mindestens eines in Wasser löslichen oder dispergierbaren Polyurethans, wie in einem der Ansprüche 1 bis 6 definiert,
b) 40 bis 99 Gew.-%, bevorzugt 50 bis 98 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 50 Gew.-% eines Treibmittels, vorzugsweise Dimethylether,
d) 0 bis 15 Gew.-% mindestens eines von a) verschiedenen, in Wasser löslichen oder dispergierbaren Haarpolymers,
e) 0 bis 0,2 Gew.-% mindestens eines wasserunlöslichen Silicons,
f) 0 bis 2 Gew.-% mindestens eines nichtionischen, siloxanhaltigen, in Wasser löslichen oder dispergierbaren Polymers.

8. Vernetzte, wasserlösliche oder wasserdispergierbare Polyurethane aus
A) mindestens einem wasserlöslichen oder wasserdispergierbaren Polyurethanpräpolymer mit endständigen Isocyanatgruppen aus
a) mindestens einer Verbindung mit einem Molekulargewicht im Bereich von 56 bis 300, die zwei aktive Wasserstoffatome pro Molekül enthält,
b) mindestens einem Polymerisat mit zwei aktiven Wasserstoffatomen pro Molekül,
c) mindestens einer Verbindung, die zwei aktive Wasserstoffatome und mindestens eine ionogene bzw. ionische Gruppe pro Molekül aufweist,
d) mindestens einem Diisocyanat,
B) mindestens einem Polymerisat mit gegenüber Isocyanatgruppen reaktiven Gruppen, die ausgewählt sind unter Hydroxyl-, sekundären Amino- und/oder Carboxylgruppen,
oder die Salze davon.

9. Polyurethane nach Anspruch 8, wobei das Verhältnis von NCO-Äquivalent der Verbindungen der Komponente d) zu Äquivalent aktives Wasserstoffatom der Komponenten a), b) und c) in einem Bereich von 1,01:1 bis 1,4:1, bevorzugt 1,03:1 bis 1,3:1, insbesondere 1,05:1 bis 1,25:1, liegt.

10. Polyurethane nach einem der Ansprüche 8 oder 9, wobei das Polymerisat B)
e) wenigstens ein α,β-ethylenisch ungesättigtes Monomer, das zusätzlich wenigstens eine gegenüber Isocyanatgruppen reaktive Gruppe pro Molekül enthält,
f) gegebenenfalls wenigstens ein α,β-ethylenisch ungesättigtes Monomer, das ausgewählt ist unter Estern α,β-ethylenisch ungesättigter Mono- und/oder Dicarbonsäuren mit C₁-bis C₂₂-Alkanolen, Amiden α,β-ethylenisch ungesättigter Mono- und/oder Dicarbonsäuren mit Mono- und Di-C₁- bis C₂₂-alkylaminen, Estern von Vinylalkohol und Allylalkohol mit C₁- bis C₄₀-Monocarbonsäuren, Vinylethern, Vinylaromaten, Vinylhalogeniden, Vinylidenhalogeniden, C₂- bis C₈-Monoolefinen, nichtaromatischen Kohlenwasserstoffen mit mindestens 2 konjugierten Doppelbindungen und Mischungen davon,
g) gegebenenfalls wenigstens ein α,β-ethylenisch ungesättigtes Monomer, das ausgewählt ist unter N-Vinylamiden, N-Vinyllactamen, primären Amiden α,β-ethylenisch ungesättigter Monocarbonsäuren, vinyl- und allylsubstituierten heteroaromatischen Verbindungen und Mischungen davon,
h) gegebenenfalls wenigstens ein weiteres Monomer mit einer radikalisch polymerisierbaren, α,β-ethylenisch ungesättigten Doppelbindung und mindestens einer ionogenen bzw. ionischen Gruppe pro Molekül,
einpolymerisiert enthält.

11. Polyurethane nach Anspruch 10, wobei das Polymerisat B)
- 0,05 bis 15 Gew.-%, bevorzugt 0,1 bis 10 Gew.-%, wenigstens einer Komponente e),
- 0 bis 99,9 Gew.-% wenigstens einer Komponente f),
- 0 bis 99,9 Gew.-% wenigstens einer Komponente g),
- 0 bis 50 Gew.-%, bevorzugt 0,1 bis 46 Gew.-%, wenigstens einer Komponente h)
einpolymerisiert enthält, wobei die Gesamtmenge der Komponenten f) und g) in einem Bereich von 30 bis 99,9 Gew.-% liegt.

12. Polyurethane nach einem der Ansprüche 10 oder 11, wobei es sich bei der Komponente e) um einen Ester der Acrylsäure oder Methacrylsäure mit einem C₂- bis C₁₂-Aminoalkohol handelt, wobei der Aminstickstoff zusätzlich einen C₁- bis C₈-Alkylrest tragen kann.

13. Polyurethane nach einem der Ansprüche 8 bis 12, wobei das Verhältnis von NCO-Äquivalent der Komponente A) zu Äquivalent aktives Wasserstoffatom der Komponente B) in einem Bereich von 20:1 bis 1:1, bevorzugt 10:1 bis 1:1, liegt.

14. Polyurethane nach Anspruch 11, wobei das Polymerisat B)
- 0,05 bis 15 Gew.-%, bevorzugt 0,1 bis 10 Gew.-% einer Komponente e), wie in Anspruch 5 definiert, vorzugsweise tert.-Butylaminoethylacrylat und/oder tert.-Butylaminoethylmethacrylat,
- 0 bis 99,9 Gew.-% wenigstens einer Komponente f),
- 0,1 bis 99,9 Gew.-% wenigstens einer Komponente g),
- 0 bis 50 Gew.-%, bevorzugt 0,1 bis 46 Gew.-%, wenigstens einer Komponente h)
einpolymerisiert enthält, wobei die Gesamtmenge der Komponenten f) und g) in einem Bereich von 30 bis 99,9 Gew.-% liegt.

15. Verwendung der Polyurethane nach einem der Ansprüche 8 bis 13 als Hilfsmittel in der Kosmetik, bevorzugt als oder in Beschichtungsmittel(n) für Haar, Haut und Nägel, insbesondere in der Haarkosmetik, als Hilfsmittel in der Pharmazie, bevorzugt als oder in Beschichtungsmittel(n) oder Bindemittel(n) für feste Arzneiformen, sowie als oder in Beschichtungsmittel(n) für die Textil-, Papier-, Druck-, Leder- und Klebstoffindustrie.

## Claims

1. Cosmetic or pharmaceutical composition, comprising at least one crosslinked, water-soluble or water-dispersible polyurethane of
A) at least one water-soluble or water-dispersible polyurethane prepolymer containing terminal isocyanate groups of
a) at least one compound having a molecular weight in the range from 56 to 300 which comprises two active hydrogen atoms per molecule,
b) at least one polymer containing two active hydrogen atoms per molecule,
c) at least one compound which contains two active hydrogen atoms and at least one ionogenic or ionic group per molecule,
d) at least one diisocyanate,
B) at least one polymer containing groups which are reactive toward isocyanate groups, chosen from hydroxyl groups, and primary and secondary amino and/or carboxyl groups,
or a salt thereof.

2. A composition as claimed in claim 1, where the ratio of NCO equivalent of the compounds of component d) to equivalent of active hydrogen atom of components a), b) and c) is in a range from 1.01:1 to 1.4:1, preferably from 1.03:1 to 1.3:1, in particular from 1.05:1 to 1.25:1.

3. A composition as claimed in either of claims 1 or 2, where the polymer B) comprises, in copolymerized form,
e) at least one α,β-ethylenically unsaturated monomer which additionally contains at least one group which is reactive toward isocyanate groups per molecule,
f) optionally at least one α,β-ethylenically unsaturated monomer which is chosen from esters of α,β-ethylenically unsaturated mono- and/or dicarboxylic acids with C₁- to C₂₂-alkanols, amides of α,β-ethylenically unsaturated mono- and/or dicarboxylic acids with mono- and di-C₁- to C₂₂-alkylamines, esters of vinyl alcohol and allyl alcohol with C₁- to C₄₀-monocarboxylic acids, vinyl ethers, aromatic vinyl compounds, vinyl halides, vinylidene halides, C₂- to C₈-monoolefins, nonaromatic hydrocarbons having at least 2 conjugated double bonds and mixtures thereof,
g) optionally at least one α,β-ethylenically unsaturated monomer which is chosen from N-vinylamides, N-vinyllactams, primary amides of α,β-ethylenically unsaturated monocarboxylic acids, vinyl- and allyl-substituted heteroaromatic compounds and mixtures thereof,
h) optionally at least one further monomer containing a free-radically polymerizable, α,β-ethylenically unsaturated double bond and at least one ionogenic or ionic group per molecule.

4. A compostion as claimed in claim 3, where the polymer B) comprises, in copolymerized form,
- from 0.05 to 15% by weight, preferably from 0.1 to 10% by weight, of at least one component e),
- from 0 to 99.9% by weight of at least one component f),
- from 0 to 99.9% by weight of at least one component g), and
- from 0 to 50% by weight, preferably from 0.1 to 46% by weight, of at least one component h)
the total amount of components f) and g) being in a range from 30 to 99.9% by weight.

5. A composition as claimed in either of claims 3 or 4, where the component e) is an ester of acrylic acid or methacrylic acid with a C₂- to C₁₂-aminoalcohol, where the amine nitrogen may additionally carry a C₁- to C₈-alkyl radical.

6. A composition as claimed in one of the preceding claims where the ratio of NCO equivalent of the component A) to equivalent of active hydrogen atom of component B) is in a range from 20:1 to 1:1, preferably from 10:1 to 1:1.

7. A composition as claimed in one of the preceding claims, comprising
a) from 0.5 to 20% by weight of at least one water-soluble or -dispersible polyurethane as defined in any of claims 1 to 6,
b) from 40 to 99% by weight, preferably from 50 to 98% by weight, of a solvent chosen from water and water-miscible solvents, preferably C₂- to C₅-alcohols, in particular ethanol, and mixtures thereof,
c) from 0 to 50% by weight of a propellant, preferably dimethyl ether,
d) from 0 to 15% by weight of at least one water-soluble or -dispersible hair polymer which is different from a),
e) from 0 to 0.2% by weight of at least one water-insoluble silicone, and
f) from 0 to 2% by weight of at least one nonionic, siloxane-containing, water-soluble or -dispersible polymer.

8. A crosslinked, water-soluble or water-dispersible polyurethane of
A) at least one water-soluble or water-dispersible polyurethane prepolymer containing terminal isocyanate groups of
a) at least one compound having a molecular weight in the range from 56 to 300 which contains two active hydrogen atoms per molecule,
b) at least one polymer containing two active hydrogen atoms per molecule,
c) at least one compound which contains two active hydrogen atoms and at least one ionogenic or ionic group per molecule,
d) at least one diisocyanate,
B) at least one polymer containing groups which are reactive toward isocyanate groups, chosen from hydroxyl groups, secondary amino groups and/or carboxyl groups,
or a salt thereof.

9. A polyurethane as claimed in claim 8, where the ratio of NCO equivalent of the compounds of component d) to equivalent of active hydrogen atom of components a), b) and c) is in a range from 1.01:1 to 1.4:1, preferably from 1.03:1 to 1.3:1, in particular from 1.05:1 to 1.25:1.

10. A polyurethane as claimed in either of claims 8 or 9, where the polymer B) comprises, in copolymerized form,
e) at least one α,β-ethylenically unsaturated monomer which additionally contains at least one group which is reactive toward isocyanate groups per molecule,
f) optionally at least one α,β-ethylenically unsaturated monomer which is chosen from esters of α,β-ethylenically unsaturated mono- and/or dicarboxylic acids with C₁- to C₂₂-alkanols, amides of α,β-ethylenically unsaturated mono- and/or dicarboxylic acids with mono- and di-C₁- to C₂₂-alkylamines, esters of vinyl alcohol and allyl alcohol with C₁- to C₄₀-monocarboxylic acids, vinyl ethers, aromatic vinyl compounds, vinyl halides, vinylidene halides, C₂- to C₈-monoolefins, nonaromatic hydrocarbons having at least 2 conjugated double bonds and mixtures thereof,
g) optionally at least one α,β-ethylenically unsaturated monomer which is chosen from N-vinylamides, N-vinyllactams, primary amides of α,β-ethylenically unsaturated monocarboxylic acids, vinyl- and allyl-substituted heteroaromatic compounds and mixtures thereof,
h) optionally at least one further monomer containing a free-radically polymerizable, α,β-ethylenically unsaturated double bond and at least one ionogenic or ionic group per molecule.

11. A polyurethane as claimed in claim 10, where the polymer B) comprises, in copolymerized form,
- from 0.05 to 15% by weight, preferably from 0.1 to 10% by weight, of at least one component e),
- from 0 to 99.9% by weight of at least one component f),
- from 0 to 99.9% by weight of at least one component g), and
- from 0 to 50% by weight, preferably from 0.1 to 46% by weight, of at least one component h)
the total amount of components f) and g) being in a range from 30 to 99.9% by weight.

12. A polyurethane as claimed in either of claims 10 or 11, where the component e) is an ester of acrylic acid or methacrylic acid with a C₂- to C₁₂-aminoalcohol, where the amine nitrogen may additionally carry a C₁- to C₈-alkyl radical.

13. A polyurethane as claimed in one of claims 8 to 12 where the ratio of NCO equivalent of the component A) to equivalent of active hydrogen atom of component B) is in a range from 20:1 to 1:1, preferably from 10:1 to 1:1.

14. A polyurethane as claimed in claim 11, wherein the polymer B) comprises, in copolymerized form
- from 0.05 to 15% by weight, preferably from 0.1 to 10% by weight, of a component e), as defined in claim 5, preferably tert-butylaminoethyl acrylate and/or tert-butylaminoethyl methacrylate,
- from 0 to 99.9% by weight of at least one component f),
- from 0.1 to 99.9% by weight of at least one component g),
- from 0 to 50% by weight, preferably from 0.1 to 46% by weight, of at least one component h)
where the total amount of components f) and g) is in a range from 30 to 99.9% by weight.

15. The use of the polyurethane as claimed in any of claims 8 to 13 as an auxiliary in cosmetics, preferably as or in coating composition(s) for hair, skin and nails, in particular in hair cosmetics, as an auxiliary in pharmacy, preferably as or in coating composition(s) or binder(s) for solid drug forms, and as or in coating composition(s) for the textile, paper, printing, leather and adhesives industries.

## Revendications

1. Produit cosmétique ou pharmaceutique contenant au moins un polyuréthanne réticulé, soluble ou dispersable dans l'eau, à base
A) d'au moins un prépolymère de polyuréthanne soluble ou dispersable dans l'eau et présentant des groupes isocyanate terminaux, à base
a) d'au moins un composé présentant un poids moléculaire de l'ordre de 56 à 300, qui contient deux atomes d'hydrogène actifs par molécule,
b) d'au moins un polymère comportant deux atomes d'hydrogène actifs par molécule,
c) d'au moins un composé qui présente deux atomes d'hydrogène actifs et au moins un groupe ionogène ou ionique par molécule,
d) d'au moins un diisocyanate,
B) d'au moins un polymère comportant des groupes qui sont réactifs vis-à-vis des groupes isocyanate et qui sont choisis parmi des groupes hydroxyle, amino primaires et secondaires et/ou carboxyle,
ou ses sels.

2. Produit suivant la revendication 1, dans lequel le rapport entre l'équivalent NCO des composés du composant d) et l'équivalent d'atome d'hydrogène actif des composants a), b) et c) est d'un ordre de 1,01/1 à 1,4/1, de préférence de 1,03/1 à 1,3/1, en particulier de 1,05/1 à 1,25/1.

3. Produit suivant l'une des revendications 1 et 2, dans lequel le polymère B) contient à l'état copolymérisé
e) au moins un monomère α,β-éthyléniquement insaturé, qui contient en supplément au moins un groupe réactif vis-à-vis des groupes isocyanate par molécule,
f) éventuellement au moins un monomère α,β-éthyléniquement insaturé, qui est choisi parmi des esters d'acides monocarboxyliques et/ou dicarboxyliques α,β-éthyléniquement insaturés avec des alcanols en C₁-C₂₂, des amides d'acides monocarboxyliques et/ou dicarboxyliques α,β-éthyléniquement insaturés avec des monoalkylamines et dialkylamines en C₁-C₂₂, des esters d'alcool vinylique et d'alcool allylique avec des acides monocarboxyliques en C₁-C₄₀, des éthers vinyliques, des substances vinyl-aromatiques, des halogénures de vinyle, des halogénures de vinylidène, des monooléfines en C₂-C₈, des hydrocarbures non aromatiques comportant au moins deux doubles liaisons conjuguées, et leurs mélanges,
g) éventuellement au moins un monomère α,β-éthyléniquement insaturé qui est choisi parmi des N-vinylamides, des N-vinyllactames, des amides primaires d'acides monocarboxyliques α,β-éthyléniquement insaturés, des composés hétéroaromatiques substitués par du vinyle et de l'allyle et leurs mélanges,
h) éventuellement au moins un autre monomère comportant une double liaison α,β-éthyléniquement insaturée, polymérisable par voie radicalaire, et au moins un groupe ionogène ou ionique par molécule.

4. Produit suivant la revendication 3, dans lequel le polymère B) contient à l'état copolymérisé
0,05 à 15% en poids, de préférence 0,1 à 10% en poids, d'au moins un composant e),
0 à 99,9% en poids d'au moins un composant f),
0 à 99,9% en poids d'au moins un composant g),
0 à 50% en poids, de préférence 0,1 à 46% en poids, d'au moins un composant h),
la quantité totale des composants f) et g) étant d'un ordre de 30 à 99,9% en poids.

5. Produit suivant l'une des revendications 3 et 4, dans lequel, en ce qui concerne le composant e), il s'agit d'un ester de l'acide acrylique ou de l'acide méthacrylique avec un aminoalcool en C₂-C₁₂, l'azote d'amine pouvant en supplément porter un radical alkyle en C₁-C₈.

6. Produit suivant l'une des revendications précédentes, dans lequel le rapport entre l'équivalent NCO du composant A) et l'équivalent d'atome d'hydrogène actif du composant B) est d'un ordre de 20/1 à 1/1, de préférence de 10/1 à 1/1.

7. Produit suivant l'une des revendications précédentes, contenant
a) 0,5 à 20% en poids d'au moins un polyuréthanne soluble ou dispersable dans l'eau, tel que défini dans une des revendications 1 à 6,
b) 40 à 99% en poids, de préférence 50 à 98% en poids, d'un solvant choisi parmi l'eau et des solvants miscibles à l'eau, de préférence des alcools en C₂-C₅, en particulier de l'éthanol, et leurs mélanges,
c) 0 à 50% en poids d'un agent gonflant, de préférence d'éther diméthylique,
d) 0 à 15% en poids d'au moins un polymère pour les cheveux, soluble ou dispersable dans l'eau, différent de a),
e) 0 à 0,2% en poids d'au moins une silicone insoluble dans l'eau,
f) 0 à 2% en poids d'au moins un polymère soluble ou dispersable dans l'eau, contenant du siloxane, non ionique.

8. Polyuréthannes réticulés, solubles ou dispersables dans l'eau, à base
A) d'au moins un prépolymère de polyuréthanne soluble ou dispersable dans l'eau et présentant des groupes isocyanate terminaux, à base
a) d'au moins un composé présentant un poids moléculaire de l'ordre de 56 à 300, qui contient deux atomes d'hydrogène actifs par molécule,
b) d'au moins un polymère comportant deux atomes d'hydrogène actifs par molécule,
c) d'au moins un composé qui présente deux atomes d'hydrogène actifs et au moins un groupe ionogène ou ionique par molécule,
d) d'au moins un diisocyanate,
B) d'au moins un polymère comportant des groupes qui sont réactifs vis-à-vis des groupes isocyanate et qui sont choisis parmi des groupes hydroxyle, amino secondaires et/ou carboxyle,
ou leurs sels.

9. Polyuréthannes suivant la revendication 8, dans lesquels le rapport entre l'équivalent NCO des composés du composant d) et l'équivalent d'atome d'hydrogène actif des composants a), b) et c) est d'un ordre de 1,01/1 à 1,4/1, de préférence de 1,03/1 à 1,3/1, en particulier de 1,05/1 à 1,25/1.

10. Polyuréthannes suivant l'une des revendications 8 et 9, dans lesquels le polymère B) contient à l'état copolymérisé
e) au moins un monomère α,β-éthyléniquement insaturé qui contient en supplément au moins un groupe réactif vis-à-vis des groupes isocyanate par molécule,
f) éventuellement au moins un monomère α,β-éthyléniquement insaturé, qui est choisi parmi des esters d'acides monocarboxyliques et/ou dicarboxyliques α,β-éthyléniquement insaturés avec des alcanols en C₁-C₂₂, des amides d'acides monocarboxyliques et/ou dicarboxyliques α,β-éthyléniquement insaturés avec des monoalkylamines et dialkylamines en C₁-C₂₂, des esters d'alcool vinylique et d'alcool allylique avec des acides monocarboxyliques en C₁-C₄₀, des éthers vinyliques, des substances vinyl-aromatiques, des halogénures de vinyle, des halogénures de vinylidène, des monooléfines en C₂-C₈, des hydrocarbures non aromatiques comportant au moins deux doubles liaisons conjuguées, et leurs mélanges,
g) éventuellement au moins un monomère α,β-éthyléniquement insaturé qui est choisi parmi des N-vinylamides, des N-vinyllactames, des amides primaires d'acides monocarboxyliques α,β-éthyléniquement insaturés, des composés hétéroaromatiques substitués par du vinyle et de l'allyle et leurs mélanges,
h) éventuellement au moins un autre monomère comportant une double liaison α,β-éthyléniquement insaturée, polymérisable par voie radicalaire et au moins un groupe ionogène ou ionique par molécule.

11. Polyuréthannes suivant la revendication 10, dans lesquels le polymère B) contient à l'état copolymérisé
0,05 à 15% en poids, de préférence 0,1 à 10% en poids, d'au moins un composant e),
0 à 99,9% en poids d'au moins un composant f),
0 à 99,9% en poids d'au moins un composant g),
0 à 50% en poids, de préférence 0,1 à 46% en poids, d'au moins un composant h),
la quantité totale des composants f) et g) étant d'un ordre de 30 à 99,9% en poids.

12. Polyuréthannes suivant l'une des revendications 10 et 11, dans lesquels, en ce qui concerne le composant e), il s'agit d'un ester de l'acide acrylique ou de l'acide méthacrylique avec un aminoalcool en C₂-C₁₂, l'azote d'amine pouvant en supplément porter un radical alkyle en C₁-C₈.

13. Polyuréthannes suivant l'une des revendications 8 à 12, dans lesquels le rapport entre l'équivalent NCO du composant A) et l'équivalent d'atome d'hydrogène actif du composant B) est d'un ordre de 20/1 à 1/1, de préférence de 10/1 à 1/1.

14. Polyuréthannes suivant la revendication 11, dans lesquels le polymère B) contient à l'état copolymérisé
0,05 à 15% en poids, de préférence 0,1 à 10% en poids, d'un composant e), tel que défini dans la revendication 5, de préférence de l'acrylate de tert-butylaminoéthyle et/ou du méthacrylate de tert-butylaminoéthyle,
0 à 99,9% en poids d'au moins un composant f),
0,1 à 99,9% en poids d'au moins un composant g),
0 à 50% en poids, de préférence 0,1 à 46% en poids, d'au moins un composant h),
la quantité totale des composants f) et g) étant d'un ordre de 30 à 99,9% en poids.

15. Utilisation des polyuréthannes suivant l'une des revendications 8 à 13, comme agents auxiliaires en cosmétique, de préférence sous la forme de ou dans des produits d'enduction pour les cheveux, la peau et les ongles, en particulier dans la cosmétique des cheveux, comme agents auxiliaires en pharmacie, de préférence sous la forme de ou dans des produits d'enduction ou des liants pour des formes médicamenteuses solides, ainsi que sous la forme de ou dans des produits d'enduction pour l'industrie des textiles, du papier, de l'impression, du cuir et des colles.
